(19) Europäisches Patentamt
European Patent Office
Office européen des brevets

(11) Publication number: 0 374 120 A2

(12) EUROPEAN PATENT APPLICATION

(21) Application number: 89870204.8

(22) Date of filing: 11.12.89

(51) Int. Cl.5: A61K 47/10, A61K 37/36, A61K 9/22, A61K 9/08, A61M 31/00

(30) Priority: 13.12.88 US 283685
18.10.89 US 421681

(43) Date of publication of application: 20.06.90 Bulletin 90/25

(84) Designated Contracting States: AT BE CH DE ES FR GB GR IT LI LU NL SE

(71) Applicant: MONSANTO COMPANY
800 North Lindbergh Boulevard
St. Louis Missouri 63167(US)

(72) Inventor: Azain, Michael Joseph
190 Rollingwood
Athens Georgia 30605(US)
Inventor: Kasser, Thomas Richard
26 Ridgecrest Drive
Chesterfield Missouri 63017(US)
Inventor: Sabacky, Milton Jerome
324 Holloway Road
Ballwin Missouri 63011(US)

(74) Representative: Lunt, John Cooper et al
Monsanto Services International S.A. Patent
Department Avenue de Tervuren 270/272
L.Box 21
B-1150 Brussels(BE)

# (54) Comosition for controlled release of polypeptides.

(57) Stable somatotropin compositions are provided that are suitable for long term administration of somatotropin by infusion devices. The composition contains at least about 10% bioactive somatotropin, an effective amount of a stabilizing polyol and a buffer to achieve a pH at which bioactivity is retained over time, such as a pH range from about 4.5 to either about 7 or the isoelectric point of the somatotropin, whichever is greater.

EP 0 374 120 A2

Xerox Copy Centre

# COMPOSITION FOR CONTROLLED RELEASE OF POLYPEPTIDES

## Background of the Invention

This invention relates to the administration of somatotropins by controlled release over an extended period of time, and more particularly to an improved composition for controlled release administration of somatotropins to animals.

A variety of methods have been proposed in the art for prolonged release of parenterally administered somatotropins. In order for such a system to be effective, it must meet several criteria. First, the rate of release must be rapid enough to provide the desired biological effect. Second, the rate of release must be slow enough to avoid overdosing the host or wasting the active protein. Third, the amount of the dose must be sufficient to provide a supply which will last over an extended period during which release is occurring at a rate adequate for the desired biological effect. Fourth, the volume of the dose must be small enough for convenient parenteral administration.

Since each polypeptide is different, e.g., in its three-dimensional structure and its interaction with other substances, the feasibility of achieving a prolonged effective release with a high loading of polypeptide in a suitable vehicle is impossible to predict. Yet in many cases, such prolonged release compositions must be developed if the biological activity of the polypeptide is to be provided in a useful, economical fashion.

One approach to providing controlled release is to formulate the somatotropin into an injectable or implantable matrix composition which retards the rate at which the polypeptide is assimilated into surrounding body fluids. Among such methods is dispersion of the somatotropin in a biocompatible oil, optionally in the presence of an anti-hydration agent (see European Patent Application Publication No. 177,478, published April 9, 1986). Somatotropin has been complexed with a water-soluble or water-dispersible carbohydrate polymer, such as dextrin, dextran, and various bean gums. This complex is administered parenterally as a solution, dispersion or paste (see European Patent Application 193,917 published September 10, 1986).

Somatotropin has also been formulated with cholesterol and compressed to form a matrix implant (see, e.g., U.S. Patent 4,452,775, to Kent, issued June 5, 1984). U.S. Patent 4,836,736, to Azain et al, which issued September 5, 1989 describes implantable pellets or tablets that are prepared by compacting somatotropin in the absence of a binder or matrix.

Another means for controlled release administration of a polypeptide is the use of a dispenser or pump containing a formulation of the polypeptide. A variety of types of infusion devides have been proposed for continuous delivery of a biologically active material. One type of implantable pump is an osmotic dispenser having an osmotic agent and a formulation of an active agent inside of a rigid semipermeable membrane, through which water is imbibed from surrounding tissue. The imbibed water causes the osmotic agent to increase in volume, forcing the active agent out of the enclosure, releasing it to be absorbed by the host. Examples of implantable osmotic dispensers include those described in U.S. Patent No. 3,995,632 to Nakano et al, U.S. Patent No. 3,845,770 to Theeuwes et al and U.S. 3,760,805 to Higuchi. The dispenser described in Nakano has one compartment at the base of the dispenser containing the osmotic agent, with the active agent in a second compartment at the top of the dispenser, with a movable barrier or piston separating the two compartments. As the osmotic agent expands, it pushes the piston into the second compartment, forcing the active agent out of an exit port. This type of arrangement will be referred to as a "push-type" implant or dispenser. The dispenser described in Higuchi has the active agent contained in a flexible bag connected to an exit port, within an enclosure containing the osmotic agent. As water is imbibed, and the osmotic agent expands, the bag is collapsed, forcing the active agent out of the exit port. This type of arrangement, in which the active agent is contained in a bag, will be referred to as a "collapsible bag type" implant or dispenser. A variety of other infusion devices have been proposed, particularly for administration of insulin. Some are implanted and some are worn externally with a cannula for infusing the active material into bodily fluids.

In any of these infusion dispensers, the active agent is typically present in a dispensable formulation that may be either aqueous or nonaqueous. In the case of somatotropins, delivery via such an infusion system presents difficulties. Somatotropins are subject to molecular modifications which can render them biologically inactive. Thus, for example, somatotropin molecules tend to bind together to form dimers and aggregates which are not bioactive. This tendency is generally accelerated by the presence of water. Moreover, it has been observed that the tendency to dimerization or aggregation is aggravated where the protein is present in high concentration in a mobil phase, such as in solution. Also, dimer and aggregate formation increases at elevated tempera tures, such as body temperature of animals into which a dispenser would be implanted. Thus, a concentrated mixture of the type that is desirable for parenteral administration normally has a maximum vulnerability to loss of bioactivity. Additionally, many protein hormones have limited solubility in water. The presence of solid particulate hormone in the formulation, or its formation by precipitation during the delivery process, may reduce hormone availability by plugging pump discharge conduits, etc. One method of avoiding these problems is to use a comparatively dilute concentration of the active polypeptide in the dispensed formulation. This approach has been used, for example, in developing formulations for administration of insulin by infusion devices. Those formulations have contained comparatively low concentrations of insulin.

The prior art reflects a number of efforts to develop formulations for delivery of protein hormones of the type that are subject to aggregation. For example, Grodsky et al U.S. Patent 4,371,523 describes the preparation of an aqueous composition containing an agent for reducing the aggregation of insulin. The anti-aggregation agent is a compound having two carboxyl moieties and at least one amine moiety, glutamic and aspartic acids being particularly preferred. This agent is mixed with insulin in an aqueous system and the pH is adjusted to about the isoelectric point of the anti-aggregation agent.

Blackshear et al U.S. Patent 4,439,181 describes mixing glycerol or another polyol with an aqueous protein hormone solution prior to introduction of the solution into a drug delivery system. The glycerol or other polyol is added in an amount sufficient to prevent precipitation of the protein during long term storage. The protein/polyol solution is injected into a pressurized drug storage reservoir of an implanted infusion pump. Working examples of the specification describe compositions containing insulin.

Hasegawa et al U.S. Patent 4,675,184 describes interferon compositions for treating viral distress. The compositions contain 15-60% of a tri or higher polyhydric sugar alcohol (preferably glycerin), an organic buffer to maintain a pH of 3-6, a conventional pharmaceutical carrier or diluent, and an effective amount of human interferon. As a stabilizer, the composition may also contain an anionic surfactant or albumin. The buffer may be a citrate, succinate, tartrate, fumarate, gluconate, oxalate or acetate. Inorganic buffers such as phosphate are said not to improve the stability of interferon.

Wigness et al PCT publication WO 85/02118 describes the use of glycerol to prevent the precipitation of proteins, such as hormone preparations, within drug delivery systems that depend on the fluidity of the infusate for proper function. A buffer, such as a phosphate buffer, is used to maintain the solution within one pH unit of the optimum pH of the protein so as to maintain bioactivity of the protein. The optimum pH is said to be 3.5 for sulfated insulin and 7.4 for standard neutral insulin. Stated objectives are to avoid the isoelectric point of insulin (5.4), which causes precipitation, and to avoid alkaline pH (greater than 8.0), which causes denaturing of the protein. Example 1 of the specification describes a composition containing 20-40 units (approximately 1-2 mg) of porcine insulin per ml, 0.2% phenol, 12mM sodium bicarbonate and 80% by volume glycerol. The compositions disclosed are adapted for delivery via an implantable infusion pump. While the specific disclosure is limited to insulin, the specification states that other infusible proteins, including growth hormone, glucagon and the like are subject to the same precipitation problems.

## Summary of the Invention

Among the several objects of the present invention may be noted the provision of a dispensable composition adapted for long term delivery of a somatotropin from an implantable dispenser such as an osmotic dispenser; the provision of such a composition which contains a substantial concentration of somatotropin; the provision of such a composition in which the somatotropin remains chemically stable and bioactive and remains physically stable and does not form precipitates over long periods of storage and which remains chemically stable and bioactive and physically stable at body temperatures for the period of time over which prolonged administration of the somatotropin is desired; and the provision of such a composition which is readily dispensable at body temperature.

Further objects of the invention include the provision of a method for preparation of the composition of the invention.

Briefly, therefore, the present invention is directed to a dispensable somatotropin composition comprising at least about 10% bioactive somatotropin, preferably a bovine or porcine somatotropin, an effective amount of a stabilizing polyol, having three hydroxyl groups, such as glycerol or tris(hydroxymethyl)-aminomethane and sufficient amounts of a buffer to achieve a pH in a range in which the somatotropin retains its bioactivity over a period of time sufficient for efficacious use of the composition for prolonged administration of the somatotropin to an animal. In one preferred embodiment, the composition exhibits a pH between about 4.5 and either about 7 or about the isoelectric point of the somatotropin, whichever is greater. Preferred buffers include histidine hydrochloride, an alkali metal phosphate buffer, the hydrochloride salt of tris(hydroxymethyl)aminomethane and a citrate buffer, or any other buffer that will achieve the desired pH. The composition may also contain a wetting agent to improve the rate at which the somatotropin is taken up in the excipient mixture.

The invention is further directed to a method for administering a somatotropin to an animal comprising infusing into the animal over a period of time, the compositions described above.

Further contemplated by the invention is a method for preparing the somatotropin compositions described above by mixing the polyol and an aqueous solution of a buffer, and optionally the surfactant, wetting the somatotropin with this excipient, until a single phase composition is produced.

## Brief Description of the Drawings

Figures 1 and 2 are schematic representations of implantable osmotically-powered infusion devices.

Figures 3, 6, 7 and 8 graphically represent the results of the rat growth studies of Examples 2, 5 and 6.

Figures 4 and 5 graphically represent the results of the study on dimer/aggregate formation of Example 3.

Figure 9 graphically represents the results of the milk production study of Example 10.

## Description of the Preferred Embodiments

Recombinantly produced somatotropins are typically isolated and purified under alkaline conditions. For this reason, aqueous solutions of somatotropins may be alkaline due to the presence of residual alkaline buffer, such as sodium bicarbonate. Such aqueous solutions have been demonstrated to possess substantial bioactivity. While dilute solutions of somatotropin can be stable at high pH, somatotropin solutions at high pH tend to exhibit at least some chemical instability, particularly at high concentrations. The somatotropin tends to form dimers and aggregates which often lack the bioactivity of the unagglomerated protein hormone.

Although it has been found that the chemical stability of somatotropins can be enhanced by lowering the pH of the aqueous system, optimal chemical stability is ordinarily achieved in a pH range relatively near to the isoelectric point, at which the solubility of the protein is at a minimum. As a consequence, solutions which have satisfactory chemical stability are typically not physically stable, i.e., the protein tends to precipitate, while solutions at a pH which maintains high solubility are typically not chemically stable, i.e., they lose bioactivity fairly rapidly with time.

In accordance with the present invention, it has been discovered that physical stability, chemical stability, and bioactivity of a dispensable somatotropin composition containing a high proportion of somatotropin can be maintained by incorporation of a substantial proportion of polyol, while buffering to maintain the pH of the system at a level where dimerization and aggregation are inhibited. Solubility of the protein hormone is achieved even at a pH in relative proximity to the isoelectric point. The compositions of the invention contain substantial proportions of somatotropin, exhibit substantial bioactivity as initially prepared, maintain that level of bioactivity even after long periods of storage, and are not prone to precipitation of the somatotropin from the liquid phase. The dispensable compositions of the invention are adapted for controlled release from an infusion dispenser, particularly from an osmotic dispenser.

Various somatotropins can be effectively formulated in accordance with the current invention. Examples of somatotropins useful with the current invention include avian somatotropins for treating chickens, turkeys and the like; aquatic somatotropins for treating fish and the like and particularly mammalian somatotropins for treating humans, cattle, swine, sheep, goats and the like. Particularly useful are bovine somatotropins and porcine somatotropins.

Because of the ability to produce substantial quantities of somatotropin, it is preferred to use recombinant DNA techniques to microbially express the somatotropin. Additionally, recombinant DNA techniques allow for production of variants, that are either similar to or different from the sequences of the naturally occurring somatotropins. The sequences for naturally occurring bovine and porcine somatotropins are given in Seeburg, et al, DNA, Vol. 2, No. 1 pp. 37-45(1983), which is incorporated herein by reference.

Examples of bovine somatotropin variants include, but are not limited to, polypeptides having the following amino acid sequences with unspecified amino acid residues being similar to the naturally occurring somatotropin:

$NH_2$-met-phe(1)-pro(2)....leu(126)....phe(190-COOH

$NH_2$-met-phe(1)-pro(2)....val(126)....phe(190)-COOH

$NH_2$-ala-phe(1)-pro(2)....val(126)....phe(190)-COOH

$NH_2$-ala-phe(1)-pro(2)....leu(126)....phe(190)-COOH

$NH_2$-phe(1)-pro(2)....leu(126)....phe(190)-COOH

$NH_2$-phe(1)-pro(2)....val(126)....phe(190)-COOH

$NH_2$-met-asp-glu-phe(1)-pro(2)....leu(126)....phe(190)-COOH

$NH_2$-met-asp-glu-phe(1)-pro(2)....val(126)....phe(190)-COOH

$NH_2$-met(4)-ser(5)....leu(126)....phe(190)-COOH

$NH_2$-met(4)-ser(5)....val(126)....phe(190)-COOH

$NH_2$-met-phe(10)......leu(126)....phe(190)-COOH

$NH_2$-met-phe(10)......val(126)....phe(190)-COOH

The first variant in the list above, with a methionyl residue at the N-terminus, and a leucyl residue at position 126 is referred to herein as methionyl bovine somatotropin or "MBS", and the third variant in the list above, with an alanyl residue at the N-terminus and a valyl residue at position 126 is referred to as alanyl-valyl bovine somatotropin or "ala-val BST" or "A-BST".

Examples of porcine somatotropin variants include, but are not limited to, polypeptides having the following amino acid sequences, with unspecified amino acid residues being similar to the naturally occurring somatotropin:

$NH_2$-ala-phe(1)....phe(190)-COOH

$NH_2$-met-phe(1)....phe(190)-COOH

$NH_2$-met(4)-pro(5)....phe(190)-COOH

$NH_2$-met-leu(6)....phe(190)-COOH

$NH_2$-met-ser(8)....phe(190)-COOH

$NH_2$-met-phe(10)...phe(190)-COOH

The first variant in the list above, with an alanyl residue at the N-terminus is referred to herein as alanyl porcine somatotropin or "APS". The second variant in the list above is referred to herein as methionyl porcine somatotropin or "MPS".

It is understood that the additional N-terminal methionyl residue on the variants described above could also be removed, either during or after expression. It is also understood that one or more amino acids of the following sequence -glu-arg-ala-tyr-ile-pro-glu- (which are numbers 32-38 of the bovine and porcine somatotropin sequences set forth above) may be deleted. This type of deletion is described in European Patent Application, Publication Numbers 282,318, and 282,319, both of which were published September 14, 1988. Other deletion variants with somatotropin activity can also be used, such as deletion of amino acids 32-45.

The somatotropins found most effective for administration via the composition of the invention are those which have an N-terminal group other than methionine. While certain N-methionyl somatotropins have shown significantly reduced bioactivity in a buffered polyol formulation, APS and A-BST are the preferred somatotropins.

The phrase "stabilizing polyol" means any polyol with three hydroxyl groups which maintains the somatotropin in a physically stable composition, i.e. the somatotropin does not precipitate to an undesirable degree over reasonable storage or administration period. Glycerol is the preferred polyol, however, other polyols may be used, such as tris(hydroxymethyl)aminomethane.

A physiologically compatible buffer is incorporated for controlling the pH which is exhibited by the composition within a range in which the somatotropin is bioactive. Generally, the pH exhibited by the solution should be between a minimum of about 4.5 or, preferably about 5, or more preferably 5.7 and a maximum of the greater of about 7 and about the isoelectric point of the somatotropin. The isoelectric point for APS is 7.6, for MPS is 7.3 and for A-BST is 8.6. These isoelectric points are for the standard monomeric forms obtained in bulk preparation of these somatotropins. Isoelectric points for other variants, other derivatives and other forms can be determined using standard techniques. For APS, the optimal pH is in the range of between about 6.1 and about 7.0 and for A-BST, the optimum pH is about 6.1 and about 7.5. Although various buffers can be used, it is preferred that the buffer be an alkali metal phosphate. To provide buffering in the desired pH range, it is particularly preferable that the buffer be comprised substantially of a monobasic phosphate such as, for example, sodium or potassium dihydrogen phosphate. Another effective buffer for controlling the pH in the desired range is a histidine hydrohalide, such as histidine hydrochloride. Additional buffers that maintain this pH range are citrate buffers and acid addition salts of tris-(hydroxymethyl)aminomethane, such as the hydrochloride salt. These tris(hydroxymethyl)aminomethane salts also contain hydroxyl groups and can act as a stabilizing polyol in some circumstances. Any other buffer that can maintain a pH in the desired range can be used.

It should incidentally be noted that direct measurement of the pH of the composition of the invention may not in all instances be practical. To provide a practical measurement, however, a small quantity such as a drop of the composition may be placed in about 10 ml of water, and the pH of the resulting mixture determined. It is believed that the actual pH of the composition is closely approximated by this measurement, but, in any event, it will be understood that the pH measured at such dilution is considered for purposes of this disclosure to be the pH exhibited by the composition itself.

In order to promote wetting of the somatotropin by the buffer/polyol excipient during preparation of the composition, a wetting agent, such as a nonionic surfactant is preferably incorporated as well. Such surfactant also inhibits foaming. A particularly preferred nonionic surfactant is a polyethoxylated sorbitan ester, such as a tri(polyoxyethylene) ester of sorbitan mono-oleate, such as that sold under the trade designation Tween 80 by ICI Americas Inc.

A major advantage of the use of a buffered polyol excipient for the somatotropin is the high loading achievable due to the high solubility of the somatotropin in the excipient liquid. Moreover, despite the high concentration of somatotropin in a composition which also contains a significant fraction of water, the pH maintained by the buffer inhibits the formation of dimers and other aggregates. Although it has not been determined whether the somatotropin is in true solution or colloidal solution, the somatotropin does not precipitate or otherwise separate from the excipient, either on standing or under the influence of the shear encountered in passage of the composition through the discharge opening of an infusion pump. The concentration of somatotropin in the composition is at least about 10% by weight, preferably at least about 15% by weight, more preferably at least about 20% by weight and even more preferably at least about 25% or even about 30% by weight. The somatotropin concentration may range as high as about 45% by weight, preferably as high as about 40% by weight, more preferably as high as about 35% by weight. The polyol concentration may be at least about 20% by weight or 25% by weight and may range as high as 80% by weight or 70% by weight or 60% by weight or 50% by weight or 40% by weight. A relatively high glycerol content additionally provides a bacteriostatic effect. It is generally considered that an excipient containing at least about 50% glycerol provides bacteriostatic effect. Preferably, the composition further comprises a wetting agent, such as a nonionic surfactant with optimum concentrations between about 0.07% or about 0.5% by weight and about 0.7% or 1% or sometimes 2% by weight of a nonionic surfactant. Except for the buffer salt, which in the case of a phosphate buffer may typically comprise 4% to 7% by weight, the balance of the composition typically is water. A preferred composition contains at least about 7% water, more preferably at least about 15% water, and even more preferably between about 25% and about 35% by weight water.

The composition is normally a clear, homogeneous single phase. The composition appears as a solid or semisolid at typical storage temperatures of about 4° C. The composition decreases in viscosity to produce a viscous liquid at the body temperature of an animal. In this way, the composition can be a dispensable composition without being a readily fluid composition at all temperatures. The variation in viscosity of the composition that occurs as temperature varies can be illustrated by the viscosity of a composition containing 33% APS, 33.5% glycerol and 33.5% 1M monosodium phosphate buffer. At 10° C this composition has a viscosity greater than 2,000,000 centipoise, at 25° C about 215,000 centipoise and at 37° C about 23,000 centipoise.

In relatively dilute compositions of the invention having a somatotropin concentration below about 25%, it is desirable to maintain a substantially constant weight ratio of somatotropin to both water and buffer, and to use proportions of polyol which increase as the concentration of somatotropin declines. For example, a 10% APS solution containing 44% glycerol has been found to be subject to some APS precipitation, but a composition containing 78% glycerol has been found to be physically stable. Preferably, at somatotropin concentrations below 25%, the weight ratio of somatotropin to water is maintained in a range of between about 0.9 and about 1.1 and the weight ratio of somatotropin to phosphate buffer is maintained in a range of between about 5.5 and about 7. Except for an optional wetting agent, it is preferred that the balance of these compositions with less than 25% somatotropin be essentially polyol.

As the concentration of somatotropin rises above 25%, the ratios of water and buffer to somatotropin preferably decline with increasing somatotropin concentration so as to maintain a polyol concentration as high as practicable. However, polyol content is limited by viscosity considerations, and the maximum polyol concentration is about 40-45% for compositions having a hormone content above 25%. Higher polyol concentrations provide a benefit in physical stability, but can result in viscosity that makes handling of the composition difficult.

Chemical stability of the compositions of the invention is maintained during storage over a range of combinations of temperature and storage time. At room temperature, for example, an optimum APS composition can be stored for six months or more without detectable loss of bioactivity. At body temperature, there is some loss of bioactivity, but the rate of loss is modest and linear with time. The compositions retain entirely adequate bioactivity for periods much longer than is required by most applications. Consequently, the compositions are useful for obtaining the known biological effects that can be achieved by administration of somatotropins. Such biological effects include increasing milk production in a lactating mammal, increasing feed efficiency, increasing growth, increasing mammary parencyma, and improving fat-to-lean ratio. The results achieved are dependent upon the amount of somatotropin administered and the lactation status and growth status of the animal (e.g. whether lactating, pregnant, etc.). The current invention can provide a controlled release persisting over a period of approximately six weeks or more, from a single administration.

In accordance with the method of the invention, an excipient liquid is prepared by mixing the polyol and the buffer. Advantageously, the buffer solution has an initial pH in the range of about 4.0 to about 4.4. Preferably, the buffer comprises an alkali metal dihydrogen phosphate, most preferably in a concentration of 0.5 to 2 M in aqueous solution prior to mixing with the polyol. The anionic surfactant is preferably incorporated into the polyol before the latter is mixed with the buffer solution. The excipient liquid is typically sterilized, such as by sterile filtration, and the sterile filtered excipient is then warmed preparatory to mixing with the somatotropin.

In one embodiment of the process of the invention, somatotropin powder is introduced into the sterilized excipient liquid. However, before the somatotropin powder is drawn into the bulk of the liquid, it is preferably wetted with the excipient. This may be done in a variety of ways. For example, mixing may take place in an agitated vessel such as a tank provided with a paddle mixer. In such circumstances, the bulk liquid is mechanically agitated at a rate sufficient to cause waves in the surface so that wetting of the powder is promoted, but below a rate which would draw air bubbles into the bulk liquid. As the powder becomes wetted at the surface of the bulk excipient, a "creamy layer" is formed in a stratum in which the wetted powder is drawn into the bulk liquid. Agitation serves further to disperse the wetted powder in the bulk liquid where it dissolves to form the composition of the invention. In another embodiment mixing can take place in a rolling bottle or drum. Any mixing method can be used, but it is preferred that minimum shear be used to minimize air entrainment. To remove any entrained gases from the composition, it can be held without agitation at an elevated temperature until entrained air has been removed.

The composition of the current invention can be administered by any type of infusion delivery device. Implantable delivery devices are preferred. Particularly preferred are osmotically driven implantable delivery devices. It is important that the somatotropin composition be protected from contamination from bodily fluids surrounding the implantable pump. This can be accomplished by containing the composition in a dispensing chamber that is effectively impermeable to the surrounding fluids.

Figures 1 and 2 illustrate 2 configurations of implantable osmotically driven dispensers.

Figure 1 represents a cross section of a push-type dispenser. In this type of dispenser, the wall of the dispenser 1 and 2 forms a rigid structure that encloses a compartment made up of two chambers 3 and 5, separated by a piston 4. The osmotic engine chamber 5 contains an osmotically active agent, such as a water soluble salt or a saturated solution of a water soluble salt; a water swellable polymer referred to as a hydrogel; or a combination of a hydrogel and a water soluble salt. Examples of osmotically active agents are given in US Patent 3,995,632 to Kakano et al and US Patent 4,327,725 to Cortese et al, which are incorporated herein by reference. It is preferred that the osmotically active agent be either a hydrogel or a mixture of a hydrogel and a water soluble salt. At least a portion of the wall 2 surrounding the osmotic engine chamber must be water permeable, to allow water to be imbibed into the osmotic engine chamber. Suitable materials for the semipermeable wall include materials known in the art as osmosis or reverse osmosis membranes, a number of which are disclosed in Cortese et al. As water is imbibed into the osmotic engine chamber, the size of this chamber increases, pushing the piston 4 into the beneficial agent chamber 3. The composition of the current invention is contained in the beneficial agent chamber, and it is pushed out of the orifice 6 as the piston advances. The piston can be made out of any suitable substance, such as a wax or an elastomer, that is deformable so that it maintains contact with the dispenser wall as it advances, to avoid contact between the somatotropin composition and the osmotic engine. The wall of the beneficial agent chamber should be impermeable or substantially impermeable to water, to protect the somatotropin composition from contamination with the surrounding bodily fluids, which may be detrimental to stability of the composition. Impermeability can be achieved by constructing the wall out of impermeable materials, by insertion of an impermeable liner or by application of an imperme able coating. Representative polymers to accomplish this include impermeable polyethylene, polytetrafluoroethylene, polycarbonate, polystyrene, polyamide, polyformaldehyde, polymelamine formaldehyde, polysulfone, styrene butadiene rubber, impermeable polyurethane, polypropylene, polyvinyl chloride, and the like.

Figure 2 represents a collapsible bag type dispenser, which is commercially available from Alza Corporation under the trade name Alzet® Osmotic Pump. The somatotropin composition 13 is contained within an impermeable flexible bag 12. This bag is within a rigid semipermeable wall 11. Between the bag and the wall is the osmotically active agent 14. As water from the surrounding bodily fluids is imbibed through the wall, the osmotically active agent increases in volume, causing the bag to collapse, and causing the somatotropin composition to be forced out of the dispensing tube 15. The assembly is held together and sealed with a seal 16 and a flange 17. The outer membrane is a cellulose ester blend and the collapsible bag is a thermoplastic hydrocarbon elastomer.

Other types of implantable osmotic dispensers and other types of infusion pumps are known to one skilled in the art.

The capacity of the dispenser, the concentration of the somatotropin in the composition of this invention and the rate of dispensing of the composition from the dispenser can all be controlled to achieve the desired rate for the desired amount of time. Preferred rates of administration depend upon the type of biological response that is desired and the breed and size of the animal. For instance, preferred rates of administration to finishing hogs ranges from about 0.5 mg of somatotropin per day to about 10 mg of somatotropin per day, and more preferably from about 1 mg to about 5 mg of somatotropin per day. Preferred rates of administration to cattle for enhancing lactation or enhancing growth or feed efficiency range from about 2 mg to about 60 mg of somatotropin per day, more preferably from about 5 mg to about 25 mg of somatotropin per day. Effective administration duration of 6 weeks or more can be achieved for finishing hogs and 10-12 weeks or more for cattle.

The following examples illustrate the invention.

## Example 1

An excipient was prepared by adding monobasic sodium phosphate monohydrate to water to produce a 1.0M solution of sodium phosphate, the pH of which ranged from 4 to 4.8. Glycerol and Tween 80 were added to this buffer solution in proportions sufficient to produce an excipient containing 1%, Tween, 49.5% glycerol and 49.5% of equivalent 1.0M sodium phosphate solution.

Using the excipient prepared as described above, a series of APS/phosphate/glycerol formulations was prepared in syringes. In the preparation of each formulation, APS powder was weighed into the barrel of the syringe with the plunger removed. A predetermined amount of the excipient was introduced into the syringe, the plunger was then inserted and most of the air forced out from the port. The syringe was then rotated at 2-3 rpm at room temperature and/or placed in a 37° C incubator for several hours. The formation of a solution was aided by the inclusion of Tween 80. Similarly, a series of formulations was prepared in a paddle mixer. The optimum mixing occurred when the mixing rate was sufficient to form a wave on the surface of the liquid, but was low enough to minimize air entrainment.

## Example 2

An aqueous APS composition was prepared containing 30% by weight APS, 33.5% by weight glycerol, 33.5% by weight water and sufficient histidine hydrochloride (about 3% by weight) to provide a composition pH of approximately 6.0. For comparative purposes, solutions were prepared respectively containing: 30% APS in water; 30% APS in water containing sufficient histidine hydrochloride buffer to provide a pH of 6.0; 30% APS in water containing sufficient histidine base to provide a pH of 8.5; and 30% APS in 50% glycerol, the overall solution thus containing 30% APS, 35% glycerol, and 35% water. Each of the solutions was stored for 4 days at 37° C, after which each was subjected to size exclusion HPLC for determination of the proportion of dimer formed, formation of large amounts of dimer indicate a lack of chemical stability. Each of the solutions was visually examined for cloudiness, indicating precipitation of the composition, and lack of physical stability. The results of these tests are set forth in Table 1.

Table 1

| | Water | His-HCl in Water | His-base in Water | 50% glycerol in Water | His-HCl in 50% glycerol |
|---|---|---|---|---|---|
| pH | 9.5 | 6.0 | 8.5 | 9.5 | 6.0 |
| % Dimer SE-HPLC | 46.7 | 3.5 | 27.4 | 36.0 | 2.1 |
| Physical Stability | + | - | + | + | + |
| Chemical Stability | - | + | - | - | + |
| + indicates that the composition showed good stability. - indicates that the composition showed poor stability. | | | | | |

The bioactivities of the APS/histidine•HCl/water and APS/histidine•HCl/glycerol/water compositions were determined by mature female rat bioassay. In accordance with the test protocol, mature female Harlan, Sprague-Dawley rats (220 g) were implanted with Alzet pumps containing the solution to be tested. The volume of solution contained in the pump was the same for each of the rats. Weight gain was then observed for the rats implanted with the pumps respectively containing the two solutions tested. The results of these tests are plotted in Figure 3. Although both solutions were chemically stable as indicated by the data of Table 1, the combination with glycerol conferred superior physical stability and greater long term bioactivity.

## Example 3

An aqueous APS composition was prepared containing approximately 30% by weight APS, 34.7% by weight glycerol, 29.9% by weight water, 0.7% by weight Tween 80, and sufficient monobasic sodium phosphate (about 4.8% by weight) to provide a composition pH of approximately 6.6. For comparative purposes, solutions were prepared respectively containing: 30% APS in water; 30% APS and 0.5M $NaH_2PO_4$ in water; and 30% APS and 1.0M $NaH_2PO_4$ in water. Each of the solutions was stored for 4 days at 37° C, after which each was subjected to size exclusion HPLC for determination of the proportion of dimer formed. Each of the solutions was also examined for cloudiness, showing lack of physical stability of the solution. The results of these tests and the pH values for the respective solutions are set forth in Table 2.

Table 2

| | Water | 0.5M $NaPO_4$ in water | 1.0M $NaPO_4$ in water | 50% 1.0M $NaPO_4$ in 50% glycerol |
|---|---|---|---|---|
| pH | 9.5 | 6.7 | 6.3 | 6.5 |
| % Dimer SE-HPLC | 46.7 | 3.3 | 3.3 | 1.4 |
| Physical Stability | + | - | - | + |

A summary of the various excipients tested, the dimer formation and the physical state of each formulation are set forth in Table 3.

Table 3

| Stability of Aqueous Formulations | | | | | |
|---|---|---|---|---|---|
| APS Load (%) | Excipient | Storage Time (hours) | Size-Exclusion HPLC (% dimer) | pH | Physical Appearance |
| 10 | Water | 0 | 0.9 | 9.5 | CS |
| 20 | " | 0 | 1.6 | 9.5 | CS |
| 30 | " | 0 | 4.1 | 9.5 | CS |
| 15 | Water | 24 | 13.3 | 9.5 | CS |
| 30 | " | 24 | 30.0 | 9.5 | CS |
| 30 | 50% glycerol | 24 | 19.2 | 9.5 | CS |
| 30 | 3% His-HCl in Water | 24 | 1.9 | 6.0 | PPT |
| 30 | 3% His-HCl Water (adj. pH) | 24 | 44.6 | 9.5 | CS |
| 30 | Water | 96 | 46.7 | 9.5 | CS |
| 30 | 3% His-HCl in Water | 96 | 3.5 | 6.0 | PPT |
| 30 | 3% His-Base in Water | 96 | 27.4 | 8.5 | CS |
| 30 | 3% His-HCl 50% Glycerol | 96 | 2.1 | 6.0 | CS |
| 30 | 1.0M $NaH_2PO_4$ | 96 | 3.3 | 6.3 | PPT |
| 30 | 0.5M $NaH_2PO_4$ | 96 | 3.3 | 6.7 | PPT |
| 30 | 50% 1.0M $NaH_2PO_4$ in 50% Glycerol | 96 | 1.4 | 6.5 | CS |
| CS = Clear solution or syrup<br>PPT = Precipitated (not physically stable) | | | | | |

## Example 4

Three formulations were prepared with 30% APS and a 50% glycerol water excipient, with sufficient monobasic or dibasic sodium phosphate to result in pH's of 6.5, 7.3 and 9.5. Each of these formulations was stored at 39°C for 14 days, and % dimer of each was determined by size exclusion chromotography. The pH 6.5 formulation had 2.9% dimer, the pH 7.3 formulation had 10.6% dimer and the pH 9.5 formulation had 73.0% dimer.

Two formulations were prepared, one having 30% APS, 5% monosodium phosphate, 35% glycerol and 30% water and having a pH of 6.4; and a second having 30% APS, 3% histidine hydrochloride, 33.5% glycerol and 33.5% water and having a pH of 6. These formulations were subjected to long term stability tests at 4°C, 22°C, 37°C, and 45°C. Formation of dimer as a function of time is plotted for the phosphate/glycerol formulation in Figure 4 and for the histidine/glycerol formulation in Figure 5. The tests demonstrated the phosphate/glycerol formulation to be superior. The histidine•HCl/glycerol formulation was effective for maintaining stability of the APS, but was not as effective as the phosphate/glycerol formulation, and became somewhat yellow over time.

## Example 5

Formulation was prepared containing 30% APS, 5% monosodium phosphate, 35% glycerol and 30% water, with a pH of 6.4. This formulation was stored at room temperature for various periods of time and tested using the rat biossay of Example 2. For comparative purposes, a control was run in which no hormone was delivered to the rats, and another control was run using freshly prepared APS/phosphate/glycerol solution. The results of these bioassays are plotted in Figure 6.

Similar bioassays were run on similar APS/phosphate/glycerol specimens which had been stored at times in the range of 4 to 5 months and at various temperatures. The results of these bioassays are plotted in Figure 7.

## Example 6

Formulations were prepared containing 1.5%, 3.0%, 6.0%, and 30% APS. The 30% APS formulation was prepared by mixing APS in an excipient that was 50% glycerol and 50% 1M monosodium phosphate/water solution. The other formulations were prepared by diluting the 30% formulation with additional glycerol. These concentrations were calculated to provide dosings of 1, 2, 4.5, and 22.5 mg/day of APS, respectively, when dispensed using an Alzet® osmotic dispenser, model 2ML4. Also, a push-type osmotic dispenser was used which delivered 1.3-1.6 mg of APS per day for 42 days. Rat growth assays were conducted on each of these systems and the results are plotted in Figure 8.

## Example 7

A formulation was prepared containing 30% APS, 3% histidine hydrochloride, 33.5% glycerol and 33.5% water, which had a pH of about 6.0. This formulation was administered to a group of 8 crossbred barrows, using a 200 µl, 14 day Alzet® osmotic dispenser, resulting in an administration rate of 3.6 mg of APS per day. The filled dispenser was implanted subcutaneously in the back of the ear, and a second dispenser was implanted 14 days later. The average daily weight gain (ADG) and average daily feed consumption (FC) were recorded for each hog, and feed efficiency (FE) was calculated as FC/ADG for each hog. A lower feed efficiency is a better feed efficiency. The averaged results for the treated hogs over the 28 day treatment period are shown in Table 4, along with the results for a similar negative control group of hogs, which received no hormone and a similar positive control group of hogs that received daily injections of 3.6 mg of APS in a 25 mM sodium bicarbonate solution at pH 9.5.

Table 4

| | Negative Control (no treatment) | Positive Control (daily injection) | Treated Hogs |
|---|---|---|---|
| Days 1-14 | | | |
| ADG (kg/d)<br>FC (kg/d)<br>FE | .64<br>2.67<br>4.22 | .67<br>1.92<br>3.02 | .60<br>1.85<br>3.27 |
| Days 15-28 | | | |
| ADG (kg/d)<br>FC (kg/d)<br>FE | .74<br>2.66<br>3.64 | .66<br>1.76<br>2.71 | .58<br>1.68<br>3.49 |
| Days 1-28 | | | |
| ADG (kg/d)<br>FC (kg/d)<br>FE | .69<br>2.66<br>3.89 | .66<br>1.84<br>2.84 | .60<br>1.75<br>3.17 |

Improved feed efficiencies and elevated levels of insulin-like growth factor I, which were observed on days 15 and 29, confirm that APS was effectively delivered over the 14 day implant period.

Example 8

Seven groups of nine crossbred barrows were treated with APS. The formulations used contained the following amounts of APS and 1M monosodium phosphate in water: 1.5% APS with 1.8% phosphate solution; 3.0% APS with 3.5% phosphate solution; 4.5% APS with 5.3% phosphate solution; 6.0% APS with 7.0% phosphate solution; 15% APS with 17.5% phosphate solution; and 30% APS with 35% phosphate solution. The balance of each formulation was glycerol. The dispensers delivered the formulation in sufficient amounts to achieve the indicated daily doses. There were two control groups of hogs, one receiving no treatment (negative control) and one receiving 2 mg/day daily injection of APS in pH 9.5 sodium bicarbonate buffer (positive control). Treatment 1 was administered in a push-type implant, with treatments 2-7 administered with 2 ml Alzet® osmotic dispensers, having a 4 week duration. Treatments 6 and 7 were reimplanted at the beginning of week 5 with a 2 week Alzet® osmotic dispenser, with APS formulations calculated to maintain the indicated daily dosage. The % APS loading and the resulting daily dosages are shown below:

| TRT # | TREATMENT DESCRIPTION |
|---|---|
| 1 | 1.3-1.4 mg/day; push-type dispenser; 30% load |
| 2 | 1 mg/day; Alzet dispenser; 1.5% load |
| 3 | 2 mg/day; Alzet dispenser; 3.0% load |
| 4 | 3 mg/day; Alzet dispenser; 4.5% load |
| 5 | 4 mg/day; Alzet dispenser; 6.0% load |
| 6 | 10 mg/day; Alzet dispenser; 15.0% load |
| 7 | 20 mg/day; Alzet dispenser; 30.0% load |

Treatment 1 was continued for 6 weeks, treatments 2-5, for 5 weeks, and treatments 6 and 7 for 6 weeks. ADG, FC and FE for these treatments are shown in Table 5, compared with the negative and positive controls.

**Table 5**

| | Negative Control | Positive Control | Treatment No. 1 | 2 | 3 | 4 | 5 | 6 | 7 |
|---|---|---|---|---|---|---|---|---|---|
| **Week 1** | | | | | | | | | |
| ADG | 1.10 | 1.27 | 1.15 | 1.00 | 1.17 | 1.22 | 1.15 | .51 | .74 |
| FC | 3.11 | 2.83 | 2.88 | 2.97 | 2.84 | 2.91 | 2.64 | 1.45 | 1.76 |
| FE | 3.07 | 2.28 | 2.60 | 3.07 | 2.44 | 2.56 | 2.35 | 2.77 | 2.44 |
| **Week 2** | | | | | | | | | |
| ADG | .81 | 1.07 | .80 | .88 | .98 | 1.13 | .88 | .76 | .84 |
| FC | 3.49 | 3.06 | 3.25 | 3.13 | 3.12 | 3.04 | 2.61 | 1.69 | 2.13 |
| FE | 4.01 | 2.95 | 4.10 | 3.78 | 3.48 | 2.91 | 3.02 | 2.30 | 2.56 |
| **Week 3** | | | | | | | | | |
| ADG | 1.02 | .97 | .97 | .93 | 1.01 | .93 | .89 | 1.01 | .85 |
| FC | 2.99 | 2.63 | 3.05 | 2.84 | 3.49 | 3.00 | 2.59 | 1.88 | 2.19 |
| FE | 3.00 | 2.78 | 3.21 | 3.21 | 3.54 | 3.48 | 3.01 | 1.89 | 2.95 |
| **Week 4** | | | | | | | | | |
| ADG | .78 | .82 | .75 | .66 | .75 | .84 | 1.02 | .80 | .81 |
| FC | 3.74 | 3.07 | 3.40 | 3.30 | 3.93 | 3.43 | 3.08 | 2.17 | 2.57 |
| FE | 4.82 | 4.64 | 5.48 | 4.72 | 6.32 | 4.25 | 3.22 | 2.69 | 2.96 |
| **Week 5** | | | | | | | | | |
| ADG | .85 | .94 | .81 | .81 | .72 | .97 | .90 | .67 | .64 |
| FC | 3.49 | 2.94 | 3.33 | 3.15 | 3.44 | 3.48 | 3.10 | 1.74 | 1.63 |
| FE | 4.06 | 3.23 | 6.13 | 4.68 | 12.85 | 3.59 | 3.59 | 2.64 | 2.43 |
| **Week 6** | | | | | | | | | |
| ADG | .55 | .91 | .83 | | | | | .61 | .48 |
| FC | 3.54 | 3.21 | 3.61 | | | | | 1.98 | 1.70 |
| FE | 7.84 | 3.73 | 4.81 | | | | | 3.94 | 2.36 |

## Table 5 (cont.)

| | Negative Control | Positive Control | Treatment No. 1 | 2 | 3 | 4 | 5 | 6 | 7 |
|---|---|---|---|---|---|---|---|---|---|
| **Weeks 1-4** | | | | | | | | | |
| ADG | .93 | 1.03 | .92 | .87 | .98 | 1.03 | .98 | .81 | .82 |
| FC | 3.33 | 2.90 | 3.14 | 3.06 | 3.35 | 3.09 | 2.73 | 1.85 | 2.19 |
| FE | 3.62 | 2.84 | 3.47 | 3.85 | 3.46 | 3.00 | 2.78 | 2.33 | 2.68 |
| **Weeks 1-6** | | | | | | | | | |
| ADG | .85 | 1.00 | .89 | | | | | .75 | .77 |
| FC | 3.39 | 2.96 | 3.76 | | | | | 1.85 | 1.96 |
| FE | 4.04 | 2.97 | 3.71 | | | | | 2.49 | 2.59 |

Treatments 1, 6 and 7 showed effective release of active APS for the full 6 weeks period of those treatments. Treatments 4 and 5 showed effective release of active APS over the 5 week period of those treatments. Treatments 2 and 3 were close to the threshold at which effects can be seen.

The stability of the various formulations used in the study was observed by incubation in a closed syringe at 37° C for 7 and 28 days. The percent dimer present in the formulations was determined by size exclusion chromatography. The results are summarized in Table 6.

Table 6

| APS % Load | % Dimer 7 Days | % Dimer 28 Days |
|---|---|---|
| 1.5 | 7.6 | 12.3 |
| 3.0 | 3.7 | 6.1 |
| 4.5 | 2.6 | 4.5 |
| 6.0 | 2.2 | 3.4 |
| 15.0 | 1.8 | 2.6 |
| 30.0 | 3.0 | 5.3 |

## Example 9

Studies were conducted on 6 groups of 9 finishing hogs comparing the effect of administration of APS at differing rates, from differing formulations, all administered with a push-type osmotic dispenser. The dispensers administered a formulation having 30% APS at a rate such that the daily dose was 2 mg/day. One formulation was 30% APS, 35% glycerol and 35% 1M aqueous monosodium phosphate. Another formulation had 15% APS. Two formulations were prepared similar to the 30%, but containing 0.07% and 0.70% Tween® 80 wetting agent. A fifth formulation contained 40% APS, 0.6% Tween 80 in a phosphate/glycerol excipient. One or two dispensers were implanted as indicated to obtain the indicated daily dosages. The treatments were:

| TRT# | TREATMENT DESCRIPTION |
|---|---|
| 1 | 2 mg/Day Phosphate Glycerol; 1 Dispenser (30% Load) |
| 2 | 3 mg/Day Phosphate Glycerol; 1 Dispenser (30% Load) and 1 Dispenser (15% Load) |
| 3 | 4 mg/Day Phosphate Glycerol; 2 Dispensers (Both 30%) |
| 4 | As 1 With 0.07% Tween 80 |
| 5 | As 1 With 0.70% Tween 80 |
| 6 | 2.8 mg/Day Phosphate Glycerol + 0.6% Tween 80; 1 Dispenser (40% Load) |

The study was conducted over a 6 week period (except for treatment 2 which was terminated after 3 weeks). ADG, FC and FE results are shown in Table 7, below, compared with a negative control group that received no APS and a positive control group that received 2 mg/day daily injections of APS in pH 9.5 bicarbonate buffer.

## Table 7

| | Negative Control | Positive Control | Treatment No. 1 | 2 | 3 | 4 | 5 | 6 |
|---|---|---|---|---|---|---|---|---|
| **Week 1** | | | | | | | | |
| ADG | .76 | 1.18 | 1.07 | .99 | 1.03 | 1.03 | 1.14 | 1.14 |
| FC | 3.14 | 3.04 | 2.42 | 2.38 | 2.25 | 2.78 | 2.62 | 2.85 |
| FE | 4.18 | 2.63 | 2.30 | 2.43 | 2.22 | 2.71 | 2.36 | 2.54 |
| **Week 2** | | | | | | | | |
| ADG | .89 | .93 | .77 | .89 | .76 | .78 | .80 | .76 |
| FC | 3.32 | 2.75 | 2.38 | 2.44 | 2.24 | 2.59 | 2.60 | 2.63 |
| FE | 3.87 | 3.03 | 3.18 | 2.98 | 3.21 | 4.16 | 3.36 | 3.63 |
| **Week 3** | | | | | | | | |
| ADG | .84 | .94 | .92 | .78 | .73 | .89 | .93 | .84 |
| FC | 3.59 | 3.10 | 2.98 | 2.81 | 2.45 | 3.08 | 3.10 | 2.90 |
| FE | 4.59 | 3.34 | 3.22 | 3.94 | 4.52 | 3.76 | 3.50 | 3.53 |
| **Week 4** | | | | | | | | |
| ADG | .74 | .85 | .65 | -- | .84 | .71 | .63 | .84 |
| FC | 3.50 | 3.21 | 3.15 | -- | 2.84 | 3.16 | 3.15 | 2.96 |
| FE | 5.05 | 3.84 | 5.17 | -- | 3.91 | 4.56 | 5.02 | 3.64 |
| **Week 5** | | | | | | | | |
| ADG | .84 | .94 | .83 | -- | 1.05 | .68 | 1.01 | .90 |
| FC | 3.61 | 3.41 | 3.30 | -- | 3.69 | 3.44 | 3.52 | 3.51 |
| FE | 4.44 | 3.69 | 4.16 | -- | 3.55 | 4.44 | 3.57 | 4.05 |

Table 7 (Cont.)

| | Negative Control | Positive Control | Treatment No. 1 | 2 | 3 | 4 | 5 | 6 |
|---|---|---|---|---|---|---|---|---|
| **Week 6** | | | | | | | | |
| ADG | .84 | .91 | .93 | -- | 1.04 | 1.01 | .87 | .95 |
| FC | 3.70 | 3.27 | 3.47 | -- | 4.05 | 3.70 | 3.60 | 3.75 |
| FE | 4.57 | 3.76 | 4.05 | -- | 3.89 | 3.82 | 4.23 | 4.13 |
| **Weeks 1-6** | | | | | | | | |
| ADG | .82 | .95 | .85 | -- | .92 | .84 | .89 | .90 |
| FC | 3.49 | 3.13 | 2.96 | -- | 2.99 | 3.08 | 3.11 | 3.11 |
| FE | 4.25 | 3.30 | 3.50 | -- | 3.24 | 3.68 | 3.51 | 3.47 |

A number of the dispensers containing 30% APS in phosphate glycerol were recovered at the end of the study, and the contents were analyzed to determine dimer/aggregate content. The dimer/aggregate content was less than 10% of the total somatotropin recovered, indicating good stability over the delivery period. At the end of three weeks and at the end of the study period, all of the phosphate/glycerol treatments (1-6) resulted in increased circulating levels of porcine somatotropin and insulin-like growth factor I, as further evidence of 42 day delivery of bioactive APS.

## Example 10

Studies were conducted to determine the effect on lactation of the administration of N-alanyl bovine somatotropin (A-BST) from a formulation of this hormone in a phosphate glycerol/excipient contained in an osmotic pump.

Thirty-seven multiparous lactating Holstein cows between 93 and 252 days postpartum, were distributed into one of five treatment groups. A-BST was administered to these cows in accordance with several different procedures. In one procedure, A-BST was solublized in 70mM sodium bicarbonate buffer and administered by either daily injection at 9.0 mg per injection or twice daily injection at 4.5 mg per injection. A-BST was administered by implantation of either 14 day type 2002 Alzet® osmotic dispensers or 28 day type 2ML4 Alzet® osmotic dispensers. The 2002 dispensers were filled with a formulation having 25% A-BST, 45.8% glycerol, 28.6% 1M monosodium phosphate and 0.6% Tween 80. Three of the 2002 dispensers were implanted biweekly for a nominal biweekly dose of 126 mg A-BST. The 2ML4 dispensers were filled with a formulation having 15% A-BST, 67.2% glycerol, 17.4% 1M monosodium phosphate, and 0.4% Tween 80. A 2ML4 dispenser was implanted at the beginning of the study period, having a nominal dose of 252 mg for the study period.

Milk production of the cows was monitored over the study period. The increase in milk production of the treated cows over the control cows (both in kg/day and as % increase) is shown in Table 8 and plotted in Figure 9.

Table 8

| | Alzet Dispenser | | | | Injections | | | |
|---|---|---|---|---|---|---|---|---|
| | 14d 2002 | | 28d 2ML4 | | 9 mg 1x/d | | 4.5 mg 2x/d | |
| Week | kg/d | % | kg/d | % | kg/d | % | kg/d | % |
| 1 | 2.7 | 8.5% | 3.5 | 11.0% | 4.2 | 13.2% | 4.1 | 12.9% |
| 2 | 6.5 | 21.6% | 6.3 | 20.9% | 8.3 | 27.6% | 7.5 | 24.9% |
| 3 | 5.3 | 18.0% | 5.3 | 18.0% | 7.4 | 25.1% | 6.4 | 21.7% |
| 4 | 6.3 | 21.9% | 4.3 | 14.9% | 6.6 | 22.9% | 4.8 | 16.7% |
| 1-4 | 5.3 | 17.6% | 5.0 | 16.6% | 6.8 | 22.6% | 5.8 | 19.3% |
| 5 | 6.2 | 21.8% | 4.9 | 17.3% | 4.4 | 15.5% | 3.2 | 11.3% |
| 1-5 | 5.5 | 18.5% | 5.0 | 16.8% | 6.3 | 21.2% | 5.3 | 17.8% |

Milk production increased rapidly in response to injected A-BST and such response was maintained during the four week period. Changes in milk production within the Alzet dispenser treatments were less rapid than in the A-BST injection group, but the magnitudes of milk response obtained during week four were similar. During week 5, milk yields remained at week four levels in the two groups in which Alzet dispensers were implanted, indicating that the A-BST remained biopotent *in vivo*, for at least 35 days.

## Example 11

A number of APS compositions were prepared using the indicated APS loading and the indicated excipient. The pH and the proportion of dimer for each composition were determined. The compositions were stored at 39° C. The proportion of dimer of each composition was determined by size exclusion HPLC after 14 days, 28 days and 45 days and the clarity of each composition was noted after 45 days. The compositions and results are shown in Table 9.

Table 9

| | | | % Dimer | | | | |
|---|---|---|---|---|---|---|---|
| Loading | Excipient | pH | Fresh | 14 Days | 28 Days | 45 Days | Clarity After 45 Days |
| 33% | 33% 1.0M $NaH_2PO_4$<br>33% glycerol<br>1% Tween 80 | 6.53 | 4.3 | 8.9 | 12.6 | 17.2 | Clear |
| 33% | 33% 1.0M $NaH_2PO_4$<br>16.5% water<br>16.5% glycerol<br>1% Tween 80 | 6.53 | 4.5 | 8.5 | 11.7 | 17.2 | Slight Cloudiness |
| 33% | 67% 1.0M tris* | 7.16 | 1.9 | 20.6 | 30.3 | 37.7 | Clear |
| 33% | 67% 2.0M tris* | 6.60 | 3.9 | 13.7 | 20.3 | 26.9 | Clear |
| 33% | 33% water | 6.34 | 3.4 | 9.6 | 14.3 | 19.6 | Clear |

* tris(hydroxymethyl)aminomethane

In view of the above, it can be seen that the composition of the current invention allows the administration of bioactive somatotropin over very extended periods of time with excellent chemical and physical stability.

As various changes could be made in the above methods without departing from the scope of the invention, it is intended that all matter contained in the above description or shown in the accompanying drawings shall be interpreted as illustrative and not in a limiting sense.

## Claims

1. A dispensable somatotropin composition comprising at least about 10% bioactive somatotropin, an effective amount of a stabilizing polyol, and sufficient amounts of a buffer to achieve a pH in a range in which the somatotropin retains its bioactivity over a period of time sufficient for efficacious use of the composition for prolonged administration of the somatotropin to an animal.

2. The composition of Claim 1 which comprises at least about 15% by weight bioactive somatotropin.

3. The composition of Claim 1 which comprises at least about 20% by weight bioactive somatotropin.

4. The composition of Claim 1 in which the somatotropin is selected from the group consisting of bovine somatotropin and porcine somatotropin.

5. The composition of Claim 4 in which the somatotropin is an N-alanyl somatotropin.

6. The composition of Claim 5 in which the somatotropin is selected from the group consisting of ala-val bovine somatotropin and alanyl porcine somatotropin.

7. The composition of Claim 1 in which the stabilizing polyol is selected from the group consisting of glycerol and tris(hydroxymethyl)aminomethane.

8. The composition of Claim 1 in which the stabilizing polyol comprises from about 20% to about 80% by weight of the composition.

9. The composition of Claim 1 in which the composition exhibits a pH of between about 4.5 and either about 7 or about the isoelectric point of the somatotropin, whichever is greater.

10. The composition of Claim 9 in which the buffer is selected from the group consisting of histidine hydrochloride, and an alkali metal phosphate buffer.

11. The composition of Claim 1 further comprising a wetting agent.

12. The composition of Claim 10 in which the wetting agent is a nonionic surfactant.

13. The composition of Claim 12 in which the nonionic surfactant is a polyethoxylated sorbitan ester.

14. A dispensable somatotropin composition comprising at least about 10% by weight of a bioactive somatotropin; from about 20% to about 80% of a stabilizing polyol; and sufficient amounts of a buffer so that the composition exhibits a pH between about 4.5 and either about 7 or about the isoelectric point of the somatotropin, whichever is greater.

15. The composition of Claim 14 in which the stabilizing polyol is glycerol.

16. The composition of Claim 14 in which the buffer is selected from the group consisting of histidine hydrochloride, and an alkali metal phosphate buffer.

17. The composition of Claim 14 in which the somatotropin is selected from the group consisting of bovine somatotropin and porcine somatotropin.

18. The composition of Claim 17 in which the somatotropin is selected from the group consisting of alaval bovine somatotropin and alanyl porcine somatotropin.

19. The composition of Claim 14 further comprising a wetting agent.

20. A dispensable somatotropin composition comprising at least about 10% by weight of a bioactive bovine or porcine somatotropin; from about 20% to about 80% glycerol; and sufficient amounts of a buffer so that the composition exhibits a pH between about 4.5 and either about 7 or the isoelectric point of the somatotropin, whichever is greater.

21. A method for administering a somatotropin to an animal, comprising infusing into the animal over a period of time a somatotropin composition comprising at least about 10% bioactive somatotropin, an effective amount of a stabilizing polyol, and sufficient amounts of a buffer to achieve a pH in a range in which the somatotropin retains its bioactivity over the period of time of the infusion.

22. A method for administering a somatotropin to an animal, comprising infusing into the animal over a period of time a somatotropin composition comprising at least about 10% by weight of a bioactive somatotropin; from about 20% to about 80% of a stabilizing polyol; and sufficient amounts of a buffer so that the composition exhibits a pH between about 4.5 and either about 7 or the isoelectric point of the somatotropin, whichever is greater.

23. A method for administering a somatotropin to an animal, comprising infusing into the animal over a period of time a somatotropin composition comprising at least about 10% by weight of a bioactive bovine or porcine somatotropin; from about 20% to about 80% glycerol; and sufficient amounts of a buffer so that the composition exhibits a pH between about 4.5 and either about 7 or about the isoelectric point of the somatotropin, whichever is greater.

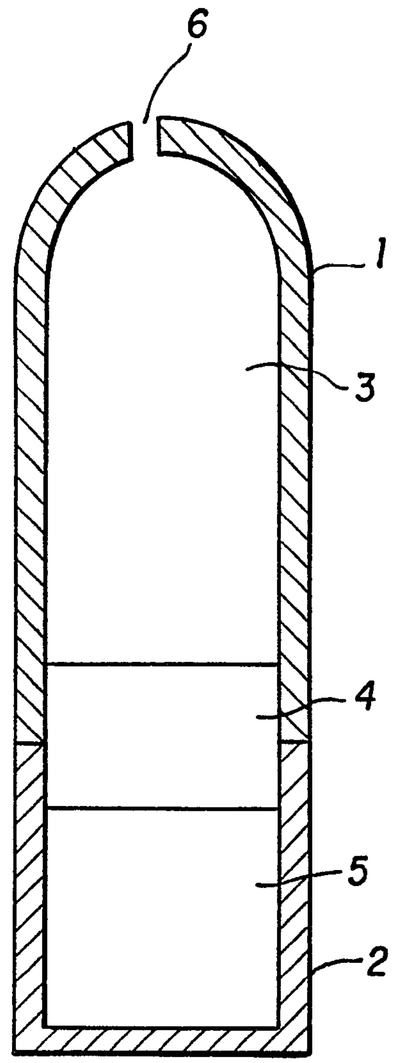


FIG. 1

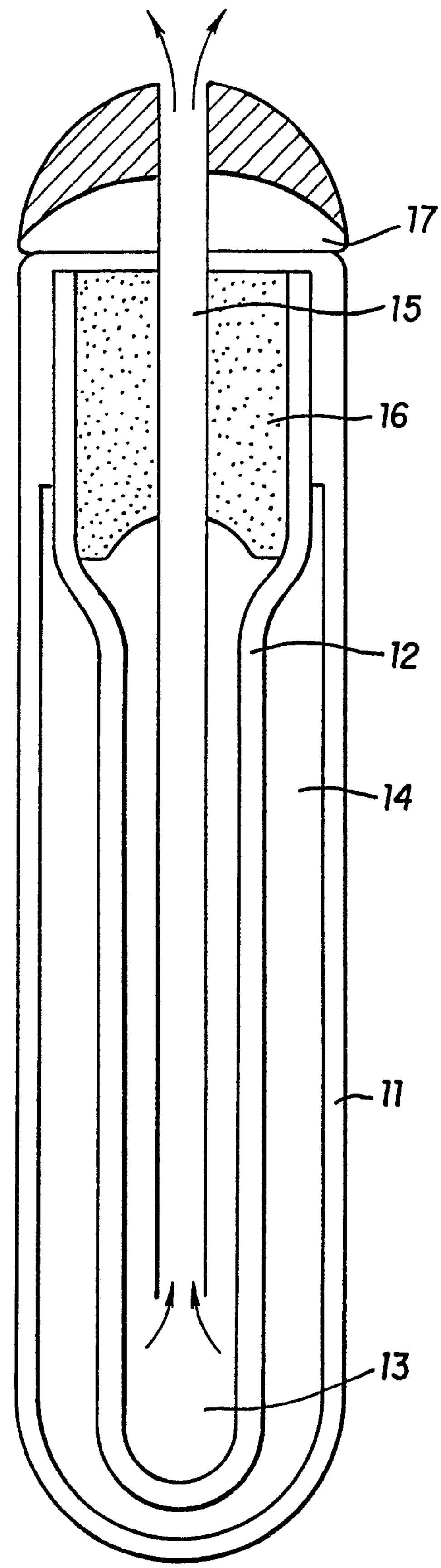


FIG. 2

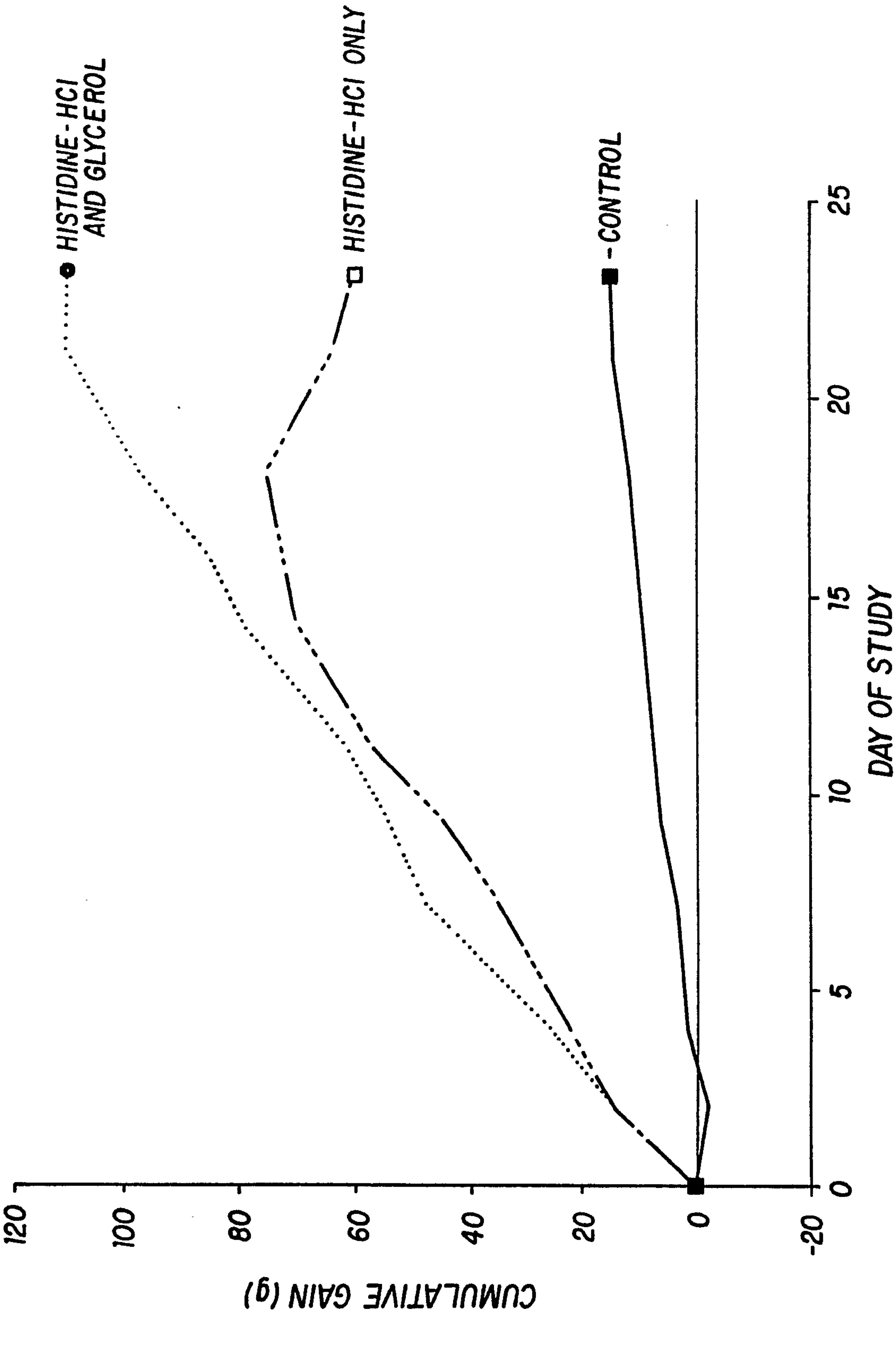
HISTIDINE-HCl AND GLYCEROL
HISTIDINE-HCl ONLY
-CONTROL
CUMULATIVE GAIN (g)
DAY OF STUDY
120
100
80
60
40
20
0
-20
0
5
10
15
20
25

FIG. 3

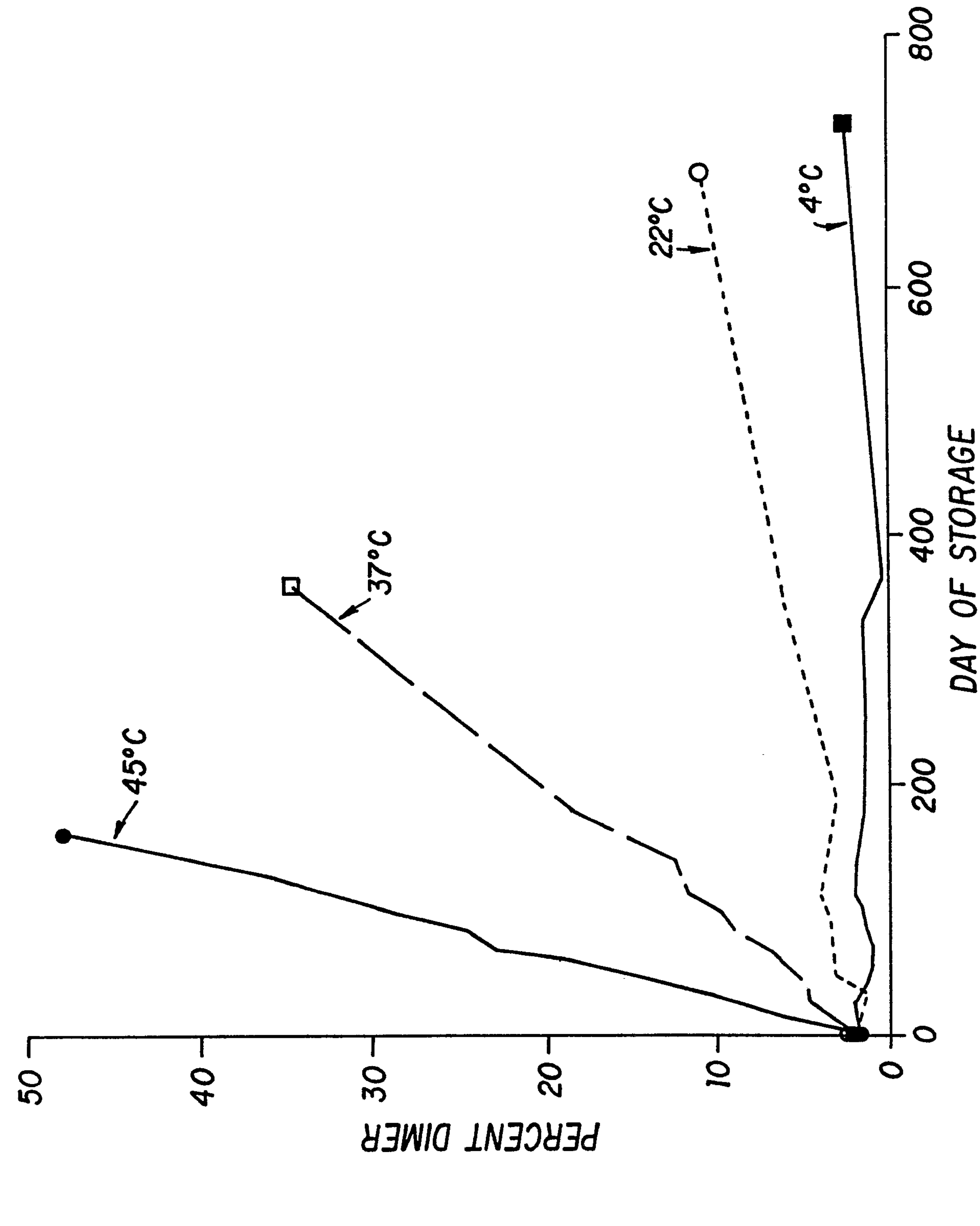
PERCENT DIMER
50
40
30
20
10
0
DAY OF STORAGE
0
200
400
600
800
45°C
37°C
22°C
4°C

FIG.4

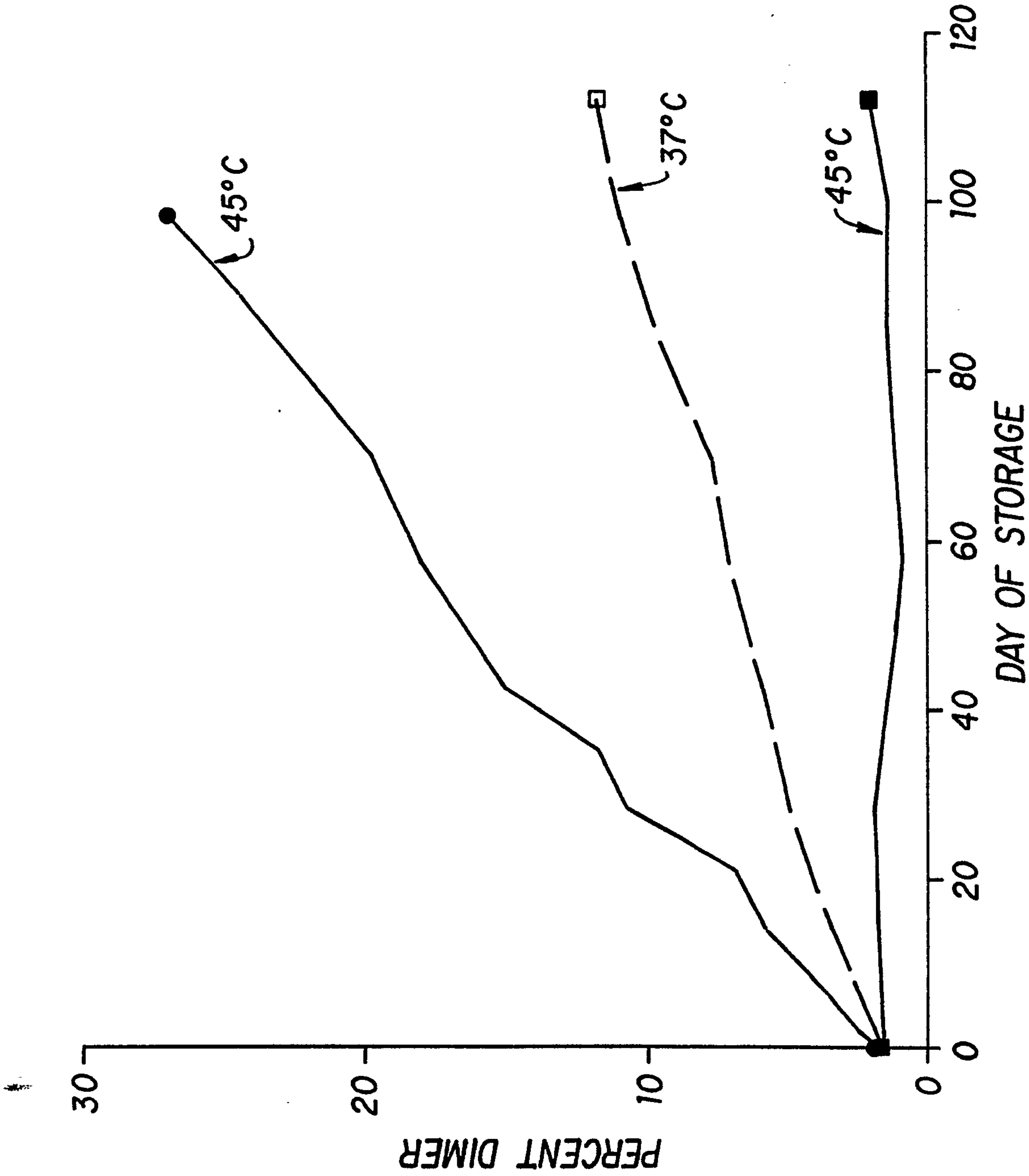
PERCENT DIMER
DAY OF STORAGE
0
10
20
30
0
20
40
60
80
100
120
45°C
37°C
45°C

FIG. 5

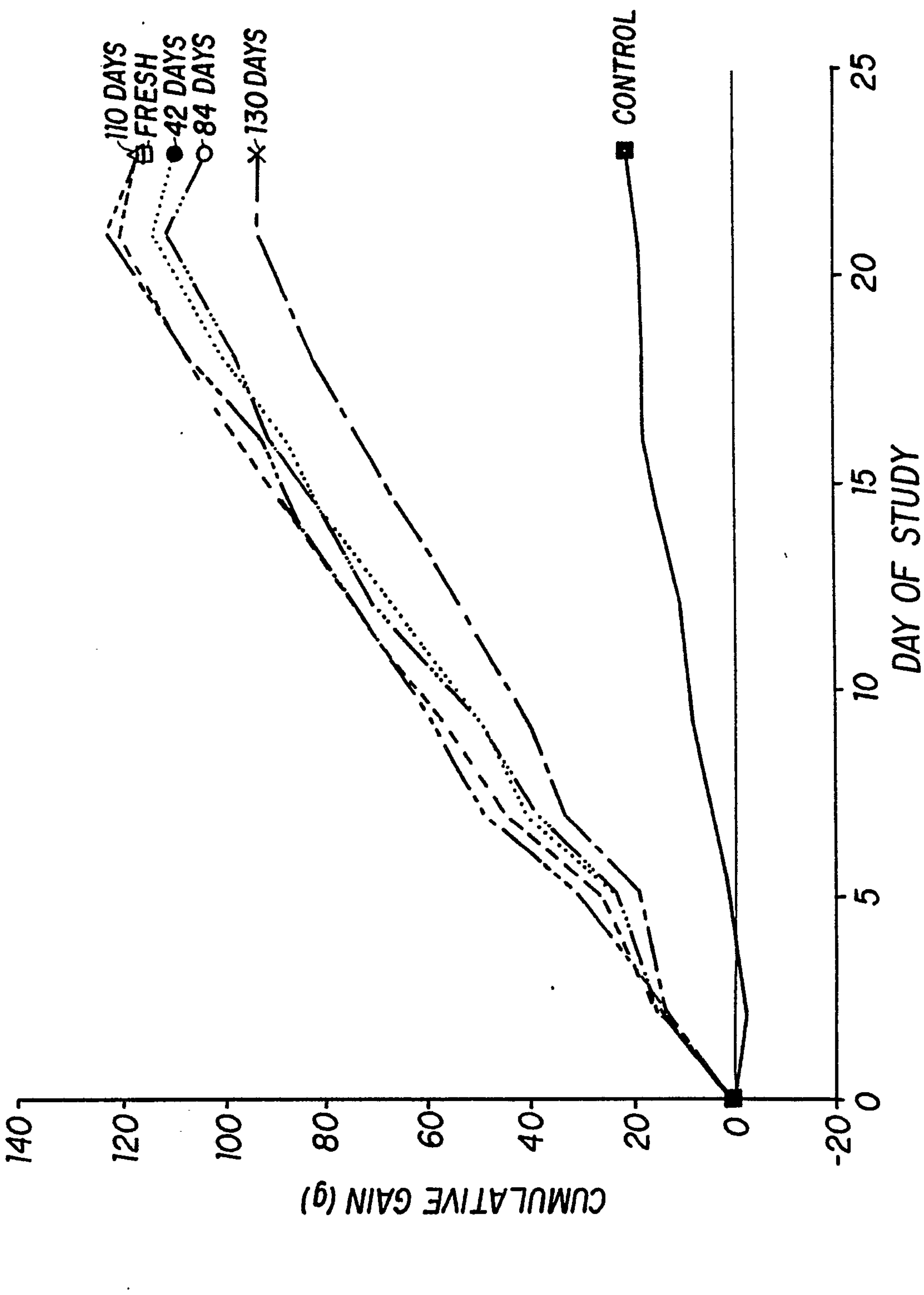
CUMULATIVE GAIN (g)
140
120
100
80
60
40
20
0
-20
0
5
10
15
20
25
DAY OF STUDY
110 DAYS
FRESH
42 DAYS
84 DAYS
130 DAYS
CONTROL

FIG. 6

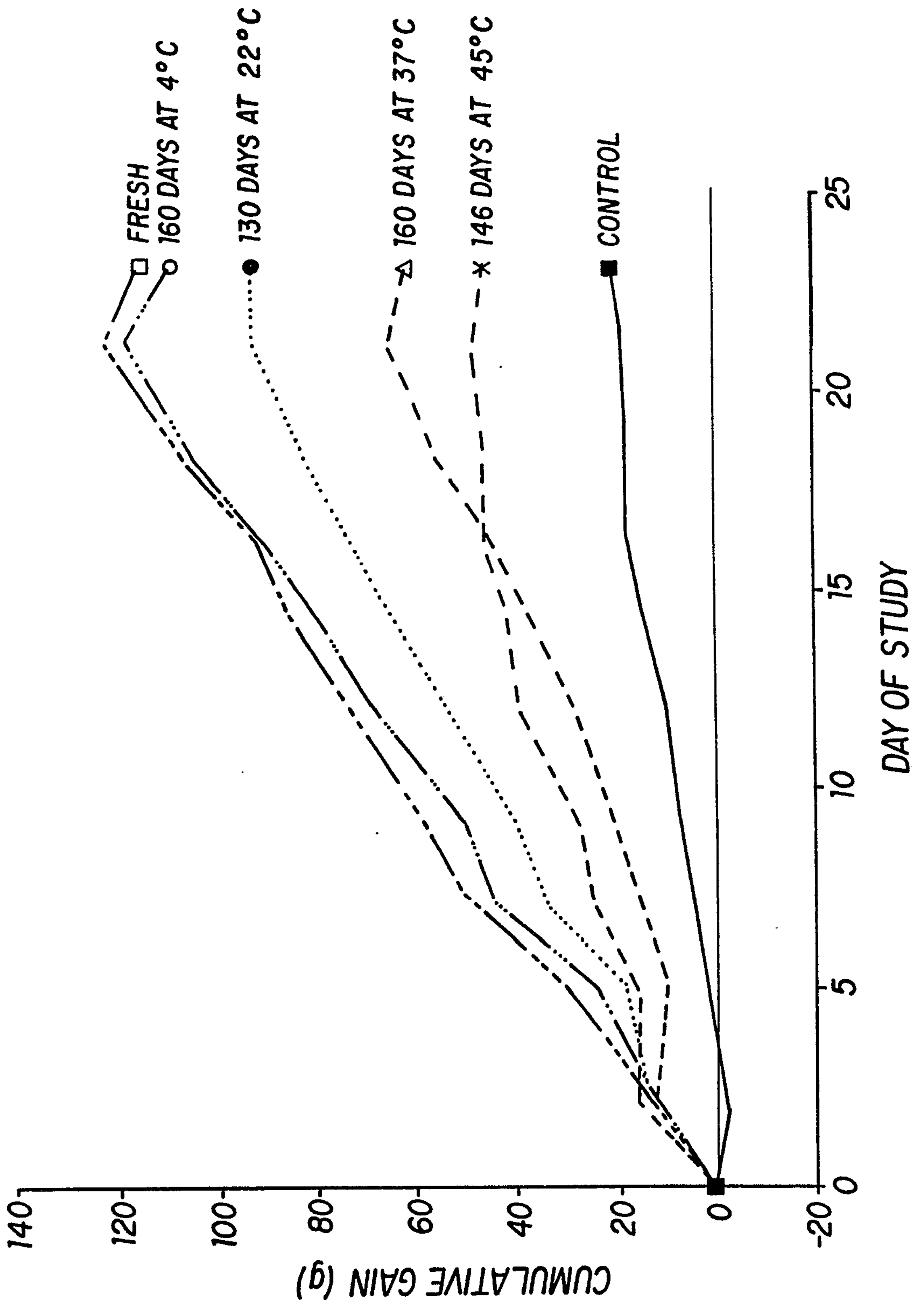
FRESH
160 DAYS AT 4°C
130 DAYS AT 22°C
160 DAYS AT 37°C
146 DAYS AT 45°C
CONTROL
CUMULATIVE GAIN (g)
140
120
100
80
60
40
20
0
-20
0
5
10
15
20
25
DAY OF STUDY

FIG. 7

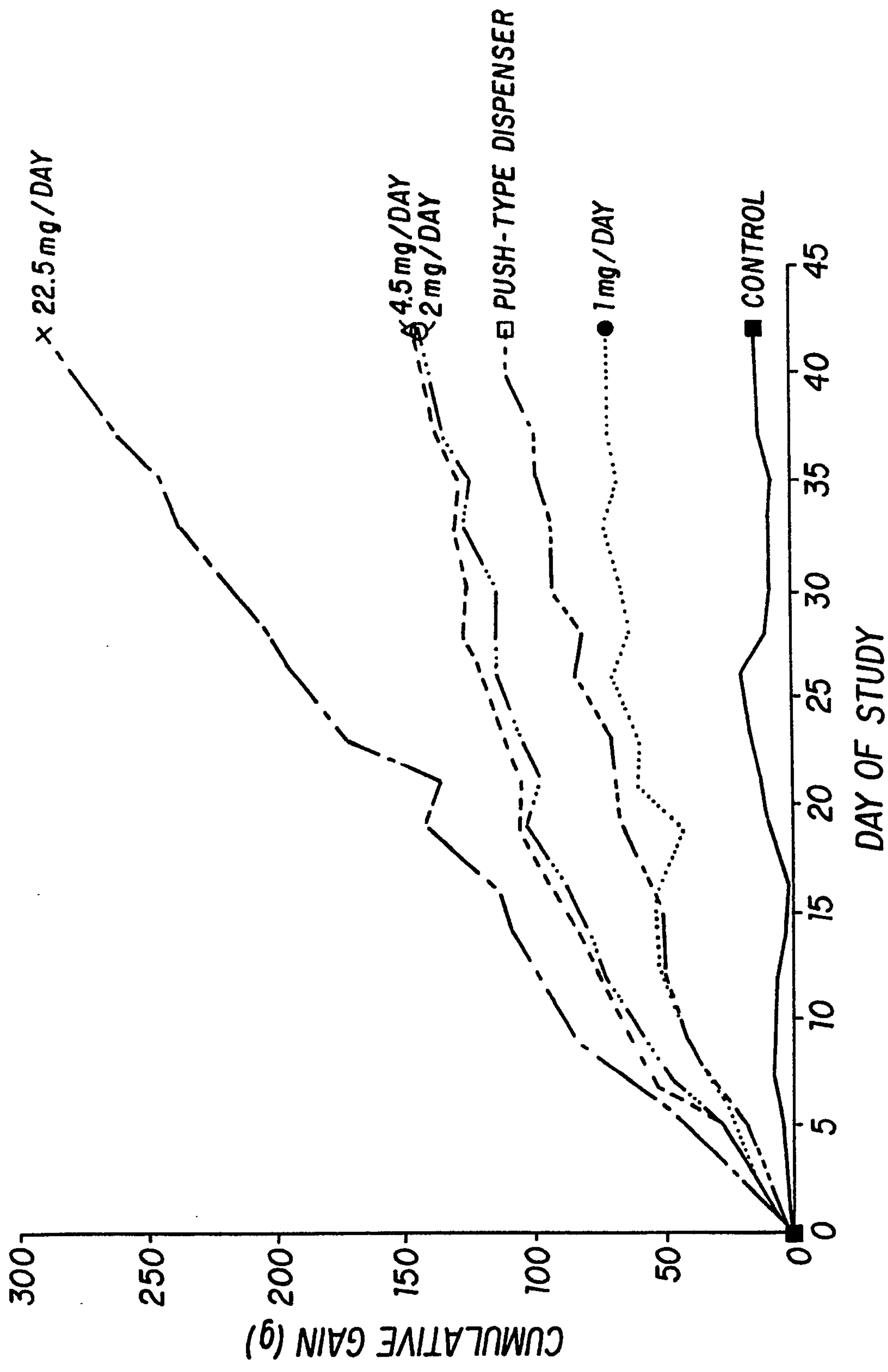

22.5 mg/DAY
4.5 mg/DAY
2 mg/DAY
PUSH-TYPE DISPENSER
1 mg/DAY
CONTROL
CUMULATIVE GAIN (g)
0
50
100
150
200
250
300
DAY OF STUDY
0
5
10
15
20
25
30
35
40
45


FIG. 8

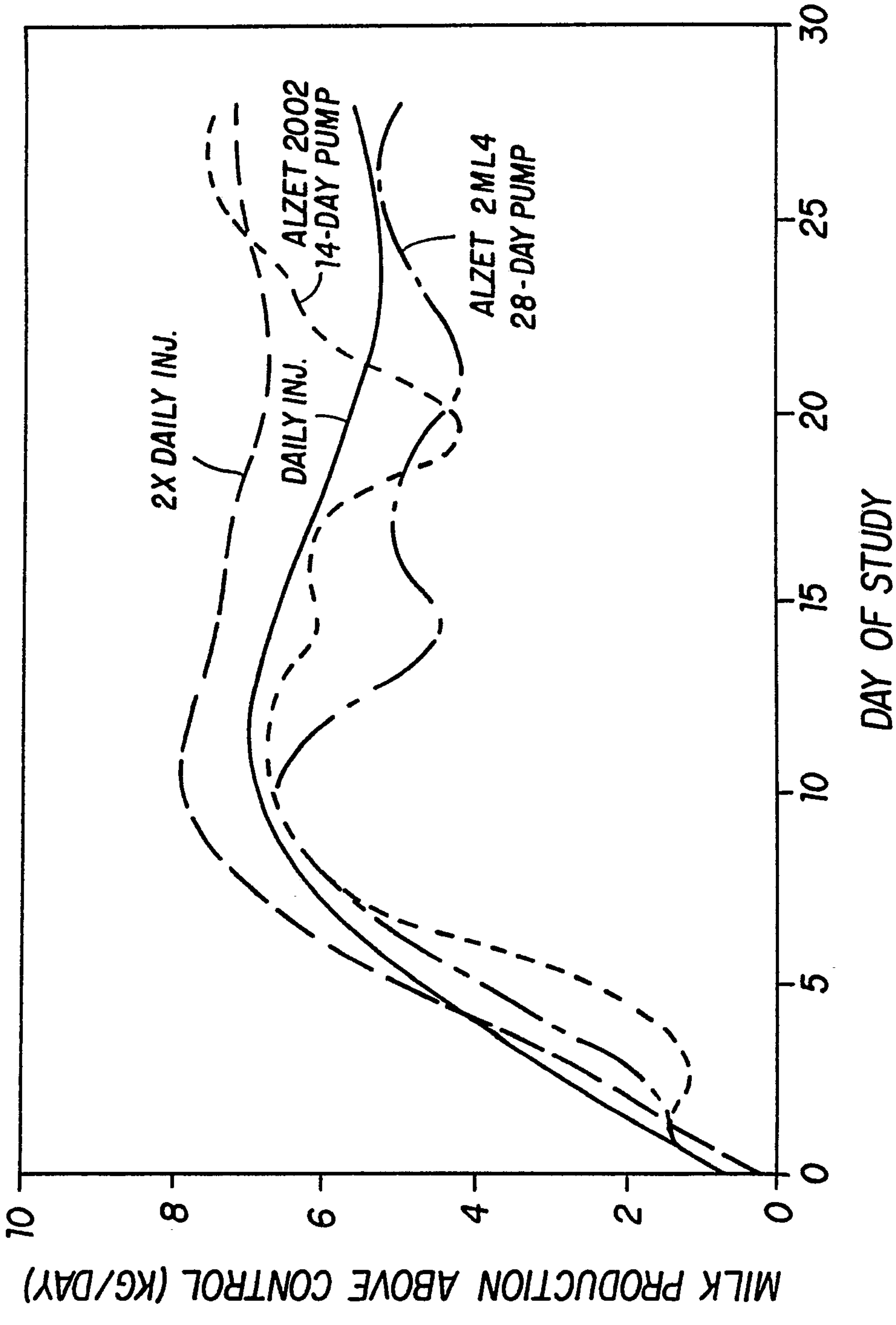
MILK PRODUCTION ABOVE CONTROL (KG/DAY)
0
2
4
6
8
10
DAY OF STUDY
0
5
10
15
20
25
30
2X DAILY INJ.
DAILY INJ.
ALZET 2002
14-DAY PUMP
ALZET 2ML4
28-DAY PUMP

FIG. 9